# EUROPEAN PATENT SPECIFICATION

(11) **EP 2 356 964 B1**
(45) Date of publication and mention of the grant of the patent: **20.06.2012**
(21) Application number: 10157526.4
(22) Date of filing: 24.03.2010
(51) Int. Cl.: A61K 8/27, A61K 8/19, A61K 8/02, A61K 8/46, A61K 8/81, A61Q 9/04, A61K 8/25

(54) **Tensionable depilatory article**
Article d'épilation pouvant être tendu
Spannbarer Enthaarungsartikel

(30) Priority: 17.02.2010 US 305199 P
(43) Date of publication of application: 17.08.2011
(73) Proprietor: The Procter & Gamble Company, Cincinnati, OH 45202 (US)
(72) Inventor: Smith, Paul, James, Whitton, Middlesex TW2 6EB (GB); Robinson, Susan, Clare, Twickenham, Middlesex TW2 6RL (GB); Dring, Neil, Charles, Medmenham, Buckinghamshire SL7 2EZ (GB); Sagel, Paul, Albert, Maineville, OH 45039 (US); Passi, Rajeev, Kumar, West Chester, OH 45069 (US); Mitra, Shekhar, Cincinnati, OH 45243 (US); Broyles, Norman Scott, Hamilton, Ohio, 45011 (US)
(74) Representative: Simpson, Kirsty Mairi

(56) References cited:
- EP-A1- 1 736 207
- WO-A1-2004/014179
- WO-A1-2006/136440
- CA-A1- 2 354 829
- DE-A1- 2 253 117
- GB-A- 1 291 377
- GB-A- 2 367 749
- JP-A- 6 135 825
- JP-A- 9 103 320
- JP-A- 2004 305 310
- US-A1- 2002 146 380
- US-A1- 2004 219 118
- DATABASE WPI Week 198819 Thomson Scientific, London, GB; AN 1988-130170 XP002592344 -& JP 63 073910 A (NITTO ELECTRIC IND CO) 4 April 1988 (1988-04-04)
- DATABASE WPI Week 200542 Thomson Scientific, London, GB; AN 2005-410646 XP2592239 & JP 2005 145895 A (KAO CORP) 9 June 2005 (2005-06-09)
- DATABASE GNPD [Online] MINTEL 01 September 2009 NN: 'Sensitive Skin Hair Removal Wax Strips' Database accession no. 1168092
- DATABASE GNPD [Online] MINTEL 01 June 2006 NN: 'In-Shower Hair Removal Cream' Database accession no. 546424

## Description

### FIELD OF THE INVENTION

The present invention relates to depilatory articles comprising a chemically active depilatory composition disposed on a substrate.

### BACKGROUND OF THE INVENTION

Depilatory compositions used to remove unwanted hair by chemical activity are known. Such compositions may comprise reducing agents to degrade keratin in the hair and thus weaken the hair strands. These compositions may take the form of creams, lotions and the like which may be applied to the unwanted hair in a variety of ways, such as with a spatula. The spatula or another suitable implement is then used to scrape off the weakened hair strands and complete the depilation process. This can be a messy and awkward procedure for the user of the depilatory cream or lotion. By disposing the depilatory composition on a material one may overcome or mitigate such disadvantages. Material-based depilatory products are known from JP63073910A, US2006002878, JP6135826A, JP11012123A JP62230711A and JP19860218477.

While addressing some of the usage problems of creams and lotions by removing the need for an application implement, known substrate-based depilatory articles do not address the problem of achieving an improved balance between ease of handling during application, and conformability to the surface of the body to which they are applied. Specifically, applicants have found that flexible substrates may collapse during handling and be difficult to accurately place on the unwanted hair, although they are able to conform to the surface of the body. Excessively rigid articles lack the capability to be conformed to the area of the body upon which there is unwanted hair, resulting in poor contact between the depilatory composition and the unwanted hair and hence an unsatisfactory hair removal process, though they may be handled with relative ease. Another problem associated with flexible substrates is that they may stretch under tension, thus altering the thickness of depilatory composition upon the substrate, which may lead to increased irritation or reduced efficacy. There exists a need, therefore, for a depilatory article that is able to provide both desirable handleability and conformability in use.

### SUMMARY OF THE INVENTION

According to a first aspect of the invention, the Applicants have surprisingly found that a depilatory article according to claim 1 meets the aforementioned need by providing a desirable balance between ease of handling during application and conformability to the surface of the body.

According to a second aspect of the invention, a cosmetic method of removing hair from the skin is provided, comprising the steps of: applying a depilatory article according to the first aspect of the invention to a surface of skin, preferably human skin, leaving said depilatory article in contact with the skin for a period of greater than 1 minute, preferably 2 to 10 minutes, more preferably 2 to 8 minutes, removing said depilatory article from the surface of the skin, and preferably rubbing, scraping, rinsing or wiping the surface of the skin in the area to which the depilatory article was applied.

According to a third aspect of the invention, a depilatory kit is also provided, comprising: a depilatory article according to the first aspect of the invention, optionally at least one of a pre-treatment skin care composition, a post-treatment skin care composition and/or a tool to assist removal of hair and/or depilatory composition after use, and packaging for said depilatory kit.

### BRIEF DESCRIPTION OF THE DRAWINGS

Fig.1. is a plan view of a depilatory article of the present invention.
Fig.2. is a side view of a depilatory article of the present invention.
Fig.3. is a side view of a depilatory article of the present invention applied to keratinous tissue.

### DETAILED DESCRIPTION OF THE INVENTION

As used herein, the term "buffering base" refers to a base capable of opposing pH changes by means of chemical or physical (solubility) processes and thereby limiting the pH to less than or equal to 13.

As used herein, the term "water impermeable" includes materials or objects through which water in its liquid state does not pass.

As used herein the term "colloid-forming" includes chemical species that are able to form stable, aqueous solid-in-liquid colloidal systems, including nano-colloidal systems.

As used herein, the term "sodium silicate" refers to Na₂SiO₃, any other silicate comprising sodium as the only cation besides silicon, and any other silicate comprising sodium. The same definition applies correspondingly to any other silicate, for example "potassium silicate" refers to K₂SiO₃, any other silicate comprising potassium as the only cation besides silicon and any other silicate comprising potassium, "ammonium silicate" to (NH₄)₂SiO₃, any other silicate comprising ammonium as the only cation besides silicon and any other silicate comprising ammonium and "manganese silicate" to Mn₂SiO₄, any other silicate comprising manganese as the only cation besides silicon and any other silicate comprising manganese.

Depilatory articles of the present invention comprise a flexible substrate, to facilitate application of the depilatory composition to keratinous tissue and prevent a messy usage experience. Depilatory articles of the present invention comprise at least two finger-tabs being substantially free of depilatory composition and positioned on substantially opposing sides of the coated region. These finger tabs enable a user to apply tension to the coated region of the flexible substrate. Surprisingly, applicants have found that applying tension across the coated region of the depilatory article creates an effect of temporarily causing the coated region to appear to exhibit an increased rigidity, enabling the user to accurately position the coated region, and hence depilatory composition on to the desired region of the body. Tensioning the coated region may be achieved in a number of ways, non-limiting examples of which include holding the depilatory article either side of the coated region, for example with the hands or a tool, so as to apply tension between the areas being held.

In a preferred embodiment, at least one finger tab extends from the perimeter of the coated region by a minimum of 1 cm, preferably from 1.5 cm to 5 cm, more preferably from 2 cm to 4 cm and even more preferably from 2.5 cm to 3.5 cm. In another preferred embodiment, both finger-tabs extend from the perimeter of the coated region by a minimum of 1 cm, preferably from 1.5 cm to 5 cm, more preferably from 2 cm to 4 cm and even more preferably from 2.5 cm to 3.5 cm, in order to aid handling of the depilatory article.

Achieving a desired dosage of depilatory composition to the surface of the skin is a further advantage of using a substrate-based product. However, if the flexible substrate is able to stretch or tear, the layer of depilatory composition disposed upon it may be thinned, thickened or rupture in places, resulting in uneven and hence less desirable depilatory activity. In particular, low depilatory efficacy may result in areas treated with thinned or ruptured areas of the composition while higher depilatory efficacy and increased irritation may result in areas treated with thickened areas of the composition.

The potential problem of a substrate stretching may be avoided by selecting a substrate that does not permanently deform during use. This problem may also be avoided by selecting a substrate with a sufficiently high secant modulus such that it is less likely to stretch during normal use. Accordingly, the flexible substrate has a secant modulus at 2% strain of greater than 1379.0 bar (20,000 psi), even more preferably greater than 2068.4 bar (30,000 psi) and even more preferably still greater than 2757.9 bar (40,000 psi) in order to achieve uniform application of the depilatory composition to the surface of the body during usage. Without wishing to be bound by theory, applicants believe that using a substrate with an excessively low secant modulus at 2% strain can deform and thus break apart the depilatory composition disposed on the flexible substrate, leading to uneven depilatory action and increased risk of irritation. The secant modulus at 2% strain may be measured readily using the American Standard Test Method (ASTM)) 'Standard Test Method for Tensile Properties of Thin Plastic Sheeting D882-09' conducted on an MTS Insight1 Tensile Tester available from MTS Systems Co, Eden Prairie, NIN, USA. This method may also be applied to non-plastic materials and is designed for use on sheets with a thickness of less than 1 mm.

The potential problem of a substrate tearing may be avoided by selecting a substrate that does not fail during usage. This problem may also be avoided by selecting a substrate with a sufficiently high nominal tensile strength such that it is less likely to tear during normal use. Accordingly, in another preferred embodiment, the flexible substrate has a nominal tensile strength of at least 5 MPa more preferably at least 10 MPa even more preferably at least 15 MPa and even more preferably still at least 18 MPa in order to achieve uniform application of the depilatory composition to the surface of the body during usage. Without wishing to be bound by theory, applicants believe that using a substrate with an excessively low nominal tensile strength can fail during usage and thus break apart the depilatory composition disposed on the flexible substrate, leading to uneven depilatory action and increased risk of irritation. The nominal tensile strength may be measured readily using the American Standard Test Method (ASTM) 'Standard Test Method for Tensile Properties of Thin Plastic Sheeting D882-09' conducted on an MTS Insight1 Tensile Tester available from MTS Systems Co, Eden Prairie, MN, USA. This method may also be applied to non-plastic materials and is designed for use on sheets with a thickness of less than 1 mm.

The flexible substrate may be water permeable or water impermeable. The flexible substrate may comprise any suitable material such as fibrous materials, papers, fabrics, non-wovens, plastics, amorphous solids, crystalline solids, foils, rubbers, latex, thermoplastic elastomers, cellular foams (open and closed cell), composites, laminates and mixtures thereof. Preferably, the flexible substrate is water impermeable. Using a water impermeable substrate prevents water loss from the depilatory composition while the depilatory composition is in contact with the keratinous tissue and thus prevents the depilatory composition from drying out. Water loss from the depilatory composition lowers the water concentration, thus increasing the concentration of active ingredients and bases present. This could result in irritation to the skin, which applicants wish to avoid.

The flexible substrate preferably comprises at least one water impermeable material and is compatible with depilatory compositions. Examples of useful water impermeable materials include but are not limited to polypropylene (PP); polyethylene (PE, including HDPE and LLDPE); polyethylene terephthalate (PET); polyvinylchloride (PVC); polyamide (PA); polycarbonate; polyurethane; cellulose acetate; polychloropene; polysulfone; polytetrafluoroethylene (PTFE); polyvinyl acetate (PVA); polystyrene; polyphenylene oxide (PPO); acrylonitrile butadiene styrene (ABS); acrylic; acrylonitrile styrene acrylate (ASA); ethylene vinyl alcohol (EVA); natural rubber, latex, nylon, nitrile, silicone and thermo plastic elastomers (TPE). The flexible substrate may comprise a single polymer or mixtures of polymers or copolymers. The flexible substrate comprises a polyolefin, preferably a polyethylene and more preferably high density polyethylene.

In an advantageous embodiment, the depilatory composition is disposed upon the water impermeable material, comprising polyolefin, preferably polyethylene and more preferably high density polyethylene. In this advantageous embodiment, there is preferably no layer of water permeable material between the depilatory composition and the water impermeable material. In a preferred embodiment, the water impermeable material forms a water impermeable layer.

The flexible substrate has a mean thickness of from 50 µm to 15 µm, preferably from 40 µm to 16 µm and more preferably from 30 µm to 17 µm.

Non-limiting examples of substrate material and thickness combinations for the flexible substrate are:

| **Substrate Material** | **Thickness [microns]** | **Rigidity [g/cm]** |
|---|---|---|
| HDPE | 13 | 0.13 |
| HDPE | 18 | 0.33 |
| HDPE | 36 | 1.05 |
| LLDPE | 23 | 0.23 |
| PP | 18 | 0.46 |

| | | |
|---|---|---|
| [HDPE is a mixture of LBI 85% M6030 and Exxon Mobil 15% LD2001 manufactured on a Merritt-Davis casting line] [LLDPE is Exxon Mobil 15% LD2001 manufactured on a Merritt-Davis casting line] [PP is Basell PH835 manufactured on a Merritt-Davis casting line] | | |

The flexible substrate may be a laminate comprising at least two materials, including non-wovens; paper; board; metal based substrates (eg aluminium foil); flocking or topical coatings (e.g. surfactants; printing); closed or open cell foams or substrates described herein above. In a preferred embodiment, at least one of the materials is water impermeable.

The flexible substrate may comprise a textured or, alternatively, micro-structured surface on at least a portion of one side. Surface texturing or micro-structuring increases the effective surface area of the flexible substrate and thus improves adherence of the depilatory composition to said substrate, facilitating an easy removal of the depilatory article by peeling it off the skin, or increases the grip of the surface, thus improving handleability. The textured structures may comprise dimples; lines or curvilinear embossments. A textured surface may be formed on the flexible substrate by any appropriate technique, including embossment calendars and casting.

The flexible substrate may be manufactured by any suitable method, including casting, injection moulding, co-injection moulding, over moulding, in-mold assembly, compression moulding, blow moulding, casting thermo or vacuum forming and where appropriate may be laminated by heat welding (which may further include the use of pressure, ultrasonic forces and radio or high frequencies), co-extrusion; adhesives, electro static adhesions (such as flocking by fibres) and topical surface applications.

Depilatory articles of the present invention comprise a depilatory composition in contact with a surface of the flexible substrate, forming a coated region. The depilatory composition may be disposed on one surface of the substrate, that surface being a depilatory surface of the depilatory article. The depilatory composition should be suitable for being placed in contact with a user's skin (and unwanted hair). Preferably, the depilatory composition is aqueous. The concentration of water in the depilatory composition is preferably at least 40%, more preferably from 50% to 98%, even more preferably still from 60% to 95% and yet more preferably from 70% to 90%, by weight of the depilatory composition. This high water level helps to improve the overall skin mildness of the depilatory composition by making it dilute, and by keeping the system more robust to pH changes, which may result in skin irritation.

Preferably, the depilatory composition is disposed upon the flexible substrate in an amount per unit area of 0.300 g/cm² to 0.001 g/cm², more preferably from 0.015 g/cm² to 0.003 g/cm², even more preferably from 0.080 g/cm² to 0.005 g/cm² and even more preferably still from 0.05 g/cm² to 0.005 g/cm², wherein the unit area refers to the coated region of the flexible substrate and not including any uncoated surface of the flexible substrate. Additionally, the area used to calculate the amount of depilatory composition disposed upon the flexible substrate is calculated ignoring any surface texturing or micro-structuring. Alternatively, the mean thickness of the depilatory composition is preferably from 0.01 mm to 3 mm, more preferably 0.1 mm to 1.5 mm, even more preferably from 0.05 mm to 0.8 mm, and even more preferably still from 0.05 mm to 0.5 mm.

A layer of depilatory composition can be applied to the flexible substrate through any known technique of applying viscous fluids to substrates, including, for example, extrusion, casting (e.g., reverse roll, knife-over roll, slot die, Gravure roll), spraying, knife blade coating, and zone coating. Such techniques may be modified to alter the quantity of depilatory composition disposed on the flexible substrate. For example, the speed at which the flexible substrate travels through an extrusion process determines the quantity of depilatory composition disposed upon said substrate. The area of depilatory composition may cover the entire surface of the flexible substrate of a portion thereof. Advantageously, the depilatory composition covers less than the entire surface of the flexible substrate to facilitate handling. The flexible substrate may comprise at least one region with two orthogonal dimensions each of a length greater than 1 cm, preferably greater than 1.5 cm and more preferably greater than 2 cm upon which no depilatory composition is disposed.

In a preferred embodiment, the depilatory composition comprises a keratin reducing agent to weaken and/or break strands of unwanted hair. Non-limiting examples of suitable keratin reducing agents include: sulphide salts such as Li₂S, Na₂S, K₂S, MgS, CaS, SrS or BaS, hydrogen sulphide salts such as NaSH or KSH, thioglycol, thioglycerol, thioglycolamide, thioglycolhydrazide, thioglycolic acid, thioglycolate salts (such as potassium thioglycolate, calcium thioglycolate, ammonium thioglycolate, diammonium dithioglycolate, glyceryl monothioglycolate, or monoethanolamine thioglycolate), thiosalicylic acid, thiomalic acid, ammonium thiolactate, monoethanolamine thiolactate, dithioerythritol, 2-mercaptopropionic acid, 1,3-dithiopropanol, glutathione, dithiothreitol, cysteine, homocysteine, N-acetyl-L-cysteine and cysteamine. Advantageously, the keratin reducing agent is present in an amount of from 0.3% to 20%, preferably from 0.8% to 15%, more preferably from 1% to 10% by weight of the composition.

Advantageously, the depilatory composition may comprise at least one thioglycolate salt or thioglycollic acid acting as a hair removal agent when the depilatory composition is applied to unwanted hair. Preferably, the depilatory composition comprises sodium, potassium, magnesium, calcium, beryllium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof. In a preferred embodiment, the concentration of the conjugate acid of the thioglycolate salt is from 0.5% to 12.0%, more preferably from 0.8% to 8.0% and even more preferably from 1.0% to 6.0% by weight of the depilatory composition.

In a preferred embodiment, the depilatory composition comprises a monovalent cation, preferably a monovalent metal cation. Without wishing to be bound by theory, the applicants believe that the presence of monovalent metal cations increases the dissociation of thioglycolate salts. The monovalent cations such as those derived from monovalent cation containing salts are able to displace the cation of the thioglycolate salt and further enhance dissociation of said thioglycolate salt. This increases the amount of deprotonated thioglycolate formed from the thioglycolate salt and therefore increases the effectiveness of the depilatory composition. Sources of monovalent cations include potassium, sodium, lithium, ammonium, tetraalkyl ammonium and imidazolium salts, which may be a component of another ingredient, for example a thickening system or skin care active. Preferred sources of monovalent cations include potassium and sodium salts.

In order to further enhance the safety of the resulting product, it is advantageous to limit the amount of monovalent cations, preferably monovalent metal cations, to which the skin is exposed when the depilatory article is used, although a small quantity may improve the efficacy of the depilatory composition. Advantageously, the quantity of monovalent cations (or monovalent metal cations in the preferred embodiment above) per unit area of the aforementioned coated region is less than 5.10 × 10⁻⁴ mol/cm², preferably less than 3 × 10⁻⁴ mol/cm⁻², more preferably from 1 × 10⁻⁹ mol/cm² to 1.5 × 10⁴ mol/cm², even more preferably from 2.50 × 10⁻⁸ mol/cm² to 6.65 × 10⁻⁵ mol/cm² and even more preferably still from 6 × 10⁻⁷ mol/cm² to 4.5 × 10⁻⁵ mol/cm². The selection of keratin reducing agent and optional ingredients including the base may be made considering the quantity of monovalent cations or monovalent metal cations achieved.

Limiting the quantity of monovalent ion present in the depilatory composition may prevent skin irritation but also limits the quantity of thioglycolate salt that may be present in a formula if monovalent ion containing thioglycolate salts or bases are used. Accordingly, in an advantageous embodiment, the depilatory composition comprises a divalent cation" preferably a divalent metal cation, and preferably wherein the thioglycolate salt, the buffering base (if present) or both comprises a divalent cation, or more preferably a divalent metal cation in order to enable the inclusion of additional depilatory active. In another preferred embodiment, the thioglycolate salt comprises a divalent metal cation. Applicants have established that thioglycolate salts comprising monovalent metal cations, such as potassium thioglycolate, are effective at removing hair from the skin, even at low doses, but may expose the skin tissue to harsh chemical conditions, resulting in irritation. On the other hand, thioglycolate salts comprising divalent metal cations, such as calcium thioglycolate, are relatively non-irritating to the skin.

In a depilatory composition comprising a mixture of monovalent and divalent ions, controlling the ratio of divalent ions to monovalent ions may also improve the safety characteristics of the depilatory articles of the present invention. Increasing the concentration of divalent ions relative to the concentration of monovalent ions increases the likelihood that any particular depilatory active species is associated with a divalent ion, rather than the more irritating monovalent ions. On the other hand, increasing the concentration of monovalent ions increases the effectiveness of the depilatory composition. Accordingly, in an alternative embodiment the ratio of the concentration of divalent ions to the concentration of monovalent ions present in the depilatory composition is advantageously in the range of from 400:1 to 0.02:1, preferably from 200:1 to 0.1:1, more preferably 60:1 to 0.3:1, even more preferably from 20:1 to 0.5:1, and even more preferably still from 15:1 to 1:1.

The pH of the depilatory composition may advantageously be in the range of from 6 to 13.8, preferably from greater than 7 to 13, more preferably from 9 to 12.9, even more preferably from 10 to 12.8, even more preferably still from 12 to 12.7 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof. Even more preferably still, the depilatory composition comprises calcium hydroxide.

In a preferred embodiment, the base is present at a concentration of from 0.1% to 10.0%, more preferably from 0.5% to 8.0% and even more preferably from 1.0% to 5.0%, by weight of the depilatory composition.

In another preferred embodiment, the depilatory composition comprises at least one silicate or silica, advantageously at least one water-soluble or colloid-forming silicate or silica.

Preferably, the depilatory composition comprises at least one water-soluble or colloid-forming silicate selected from lithium silicates; sodium silicates (including disodium metasilicate pentahydrate and disodium metasilicate nanohydrate); potassium silicates; calcium silicates, ammonium silicates; manganese silicates; imidazolium silicates, synthetic and natural silicates (clays) or mixtures thereof. More preferably, the depilatory composition comprises at least one water-soluble or colloid-forming silicate selected from synthetic clays; sodium silicates, potassium silicates, or mixtures thereof and even more preferably the depilatory composition comprises a sodium silicate or mixtures of sodium silicates.

Alternatively, the depilatory composition comprises a form of silica that is colloid-forming, (such as amorphous microporous silica), forms sol or gel systems, (such as silica gels and nano-colloidal silicas), or is mesostructured. Surface modification of silica may be advantageous to promote the formation of stable colloid systems.

Suitable synthetic and natural silicates (clays) are available commercially as: Laponite® RDS; XLS and S etc. (available from RockWood Additives Limited); Wyoming Bentonite; Californian Hectorite; Jadeite; Enstaite and Rhodonite; Benonate® EW (available from Rheox Inc.); Bentolite® (available from Southern Clay Products Inc.) Optigel® (available from Süd Chemic Rheologicals)

The silicate or silica is preferably present in the depilatory composition in an amount per unit area of the coated region of from 2.05 × 10⁻⁸ mol/cm² to 1.23 × 10⁻⁴ mol/cm², preferably from 1.64 × 10⁻⁷ mol/cm² to 3.69 × 10⁻⁵ mol/cm² and more preferably from 4.92 × 10⁻⁷ mol/cm² to 8.20 × 10⁻⁶ mol/cm². Within the preferred ranges, the effectiveness of the depilatory composition is further increased while irritation is maintained within an acceptable level. Without wishing to be bound by theory, applicants believe that an amount of silicate or silica is required in order to enhance the dissociation of the thioglycolate salt sufficiently for the increase in efficacy to be clearly apparent to the user, but that excessive dosage of silicate or silica may lead to over-dissociation of the thioglycolate salt resulting in increased skin irritation. Alternatively, the silicate or silica may be present in the depilatory composition in an amount of from 0.01 % to 5%, preferably 0.1% to 4%, more preferably 0.2% to 3% and even more preferably from 0.5% to 2% by weight of the depilatory composition.

The depilatory composition may optionally comprise a thickening agent. A representative but not exhaustive list can be found in "The Encyclopaedia of Polymers and Thickeners for Cosmetics" compiled and edited by Robert Y. Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the depilatory composition. It may be desirable to utilize gel network structures or oil-in-water emulsions to thicken the depilatory compositions. Suitable materials for preparing the gel network structures or oil-in-water emulsions are well represented in the art and include fatty materials such as fatty alcohols (for example cetyl alcohol and stearyl alcohol) alone or used in conjunction with non-polar oils such as paraffin or mineral oils. An appropriate emulsifier may also be used to form and stabilize the bilayer structure characteristic of gel network structures or to form and stabilize an oil-in-water emulsion. The thickening agent may be present at a level of from about 0.01% to about 20%, preferably from about 0.1% to about 10%, more preferably from about 0.3% to about 5%, and even more preferably from about 0.5% to about 4%, by weight of the aqueous depilatory composition.

Advantageously, the thickening agent comprises carrageenan. The carrageenan is preferably present in an amount of from 0.1 % to 10%, more preferably from 0.5% to 8%, even more preferably from 1% to 5% and even more preferably still from 2% to 4% by weight of the depilatory composition. The carrageenan may be iota, kappa or lambda carrageenan, and in a preferred embodiment is iota carrageenan. Without wishing to be bound by theory, the applicants believe that a depilatory composition comprising carrageenan has both an affinity to the surface of the skin, providing an effect analogous to a frictional resistance opposing spreading of the composition and cohesive forces that further prevent spreading and additionally prevent rupturing of the composition.

The rheological properties of the depilatory composition may also lead to improved performance in use. In particular, the yield point describes the resistance of the depilatory composition to deformation under environmental stress. If the yield point is too high, then the depilatory composition may not deform sufficiently, with hair fibres unable to enter the depilatory composition effectively upon application, resulting in less desirable depilatory effectiveness. If the yield point is too low, however, then the depilatory composition may flow during storage, transport or use and is not cleanly removed from the skin upon removal of the depilatory article, thus requiring the inconvenience of additional wiping and risking irritation to the user. Accordingly, the phase angle of the depilatory composition preferably has a yield point from 10 Pa to 2000 Pa, more preferably from 30 Pa to 1200 Pa, even more preferably from 45 Pa to 500 Pa and even more preferably still from 60 Pa to 300 Pa, when measured via a stress controlled amplitude sweep at a frequency of 1 Hz and a temperature of 25°C. The yield point described is defined as the 5% decrease in magnitude of the elastic modulus G' linear viscoelastic plateau value as measured on a TA1000 Rheometer, available from TA Instruments of New Castle, Delaware, USA. The rheological properties of the depilatory composition may be altered by changing the concentration or identity of the thickening system and the water content of the depilatory composition.

Advantageously, the depilatory composition displays an elastic modulus G' which exceeds its viscous modulus G" at all frequencies below 60 rad/s, preferably below 20 rad/s, more preferably below 10 rad/s and even more preferably below 1 rad/s; when measured via a strain controlled frequency sweep; at a strain of 1% and a temperature of 25°C. The elastic modulus of the depilatory composition exceeds its viscous modulus at a low frequency of applied stress. This indicates that the depilatory composition is behaving in a solid-like manner at rest and is of particular benefit when the depilatory composition is interposed between two substrates, for example a substrate and a protective release layer.

In another preferred embodiment, the depilatory composition displays a high degree of shear thinning behaviour enabling the effective coating of target hairs during application and improve depilatory efficacy. Accordingly, at a low shear rate of 0.1 s⁻¹, the dynamic viscosity of the depilatory composition is preferably 1000 Pa.s to 10000 Pa.s measured at a temperature of 25°C, whereas at a high shear rate of 1000 s⁻¹, the dynamic viscosity of the depilatory composition is preferably 0.1 Pa.s to I Pa.s, measured at a temperature of 25°C.

The depilatory composition may also include other skin care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted for fine lines, wrinkles and uneven skin tone including retinoids), cosmetic compositions; anti-inflammatory agents (including corticosteroids); anti-oxidants (including flavonoids) radical scavengers; sunscreen agents; skin cooling or warming agents and the like. The depilatory composition may comprise one or more skin care ingredients present in an amount of from about 0.001% to about 10%, more preferably from about 0.01% to about 7%, and even more preferably from about 0.025% to about 5%, by weight of the depilatory composition.

An accelerant may be employed in the depilatory composition. This optional component accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the depilatory composition.

The depilatory composition may further comprise components known, conventionally used, or otherwise effective for use in hair removal compositions particularly dyes; pigments (including ultra marines and talc); anionic, cationic, non-ionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

Depilatory articles of the present invention may take any form suitable for applying to keratinous tissue. The size and shape of the depilatory article may take any form suitable for application to the body area from which hair is to be removed. The depilatory article will preferably relate to the body area or zone from which hair is to be removed, especially the face (including the jaw, chin and upper lip regions of the face), underarm and bikini areas. Preferably, the depilatory article takes the form of a mask (configured for the face) or a strip/patch (configured for general use). In another preferred embodiment, the flexible substrate of the depilatory article is substantially planar.

The coated region preferably comprises an upper-lip portion adapted to be placed above a human mouth, and a first return portion projecting from the upper lip portion and adapted to be placed contiguously with the outer extremity of the vermilion lip in a first corner of the mouth. The return portion has a length along its greatest dimension of at least 0.2 cm, preferably from 0.5 cm to 5 cm, more preferably from 0.75 cm to 4 cm, even more preferably from 1 cm to 3 cm. Applicants have found that this configuration enables the user to remove unwanted hair from the skin immediately surrounding the corner of the mouth while lowering the risk of depilatory composition contacting the vermillion lip, where it may cause irritation. In an alternative embodiment, the coated region further comprises a second return portion projecting from the upper lip portion and adapted to be placed contiguously with the outer extremity of the vermillion lip in a second corner of the mouth.

Advantageously, the upper lip portion has a length along its greatest dimension of at least 0.2 cm, preferably from 0.5 cm to 15 cm, more preferably from 1 cm to 12 cm, even more preferably from 2 cm to 10 cm and even more preferably still from 3 cm to 8 cm. This dimension enables the upper lip portion to cover a desirable length of the upper lip and thus achieve the desired depilatory action. In a preferred embodiment, the upper lip portion is adapted to be placed to be at least partially contiguously with the upper border of the upper vermilion lip, to enable depilatory action to be achieved on the skin immediately surrounding the upper vermilion lip while lowering the risk of depilatory composition contacting the upper vermilion lip, where it may cause irritation.

In another preferred embodiment, the coated region comprises a lower lip portion adapted to be placed below a human mouth, preferably wherein the lower lip portion is adapted to be placed to be least partially contiguously with the lower border of the lower vermilion lip to enable depilatory action to be achieved on the skin immediately surrounding the lower vermilion lip while lowering the risk of depilatory composition contacting the lower vermilion lip, where it may cause irritation.

Depilatory articles of the present invention may comprise at least two finger-tabs being substantially free of depilatory composition and positioned on substantially opposing sides of the coated region. These finger tabs enable a user to apply tension to the coated region of the flexible substrate. Surprisingly, applicants have found that applying tension across the coated region of the depilatory article creates an effect of temporarily causing the coated region to exhibit an apparent increased rigidity, enabling the user to accurately position the coated region, and hence depilatory composition on to the desired region of the body. Tensioning the coated region may be achieved in a number of ways, non-limiting examples of which include holding the depilatory article either side of the coated region, for example with the hands or a tool, so as to apply tension between the areas being held. Alternatively, depilatory articles of the present invention may comprise at least one finger-tab being substantially free of depilatory composition and positioned to allow the weight of the article to tension the coated region when being held by the finger-tab.

In a preferred embodiment, at least one finger tab extends from the perimeter of the coated region by a minimum of 1 cm, preferably from 1.5 cm to 5 cm, more preferably from 2 cm to 4 cm and even more preferably from 2.5 cm to 3.5 cm. In another preferred embodiment, both finger-tabs extend from the perimeter of the coated region by a minimum of 1 cm, preferably from 1.5 cm to 5 cm, more preferably from 2 cm to 4 cm and even more preferably from 2.5 cm to 3.5 cm, in order to aid handling of the depilatory article.

Depilatory articles of the present invention may comprise a protective release layer removably attached to the depilatory composition, preferably on a surface of the depilatory composition substantially opposing that which is in contact with the flexible substrate. The protective release layer may comprise materials including polymer resins such as a polyolefins e.g. polypropylene (including stratified biaxially oriented polypropylene (SBOPP)), polyethylene (including LDPE; LLDPE; HDPE; Metallocene) or polyethylene terephthalate. Alternative materials which may be used include polyvinylchloride, polyamide, acetyl, acrylonitrile butadiene styrene, acrylic, acrylonitrile styrene acrylate, ethylene vinyl alcohol, ethylene vinyl acetate, Nylon, Latex, natural or synthetic rubbers, polycarbonate, polystyrene, silicone or thermo plastic elastomer, thermo plastic vulcanate or copolymers of said materials. Where appropriate the protective release layer may comprise one or more laminations, combinations of multiple layers and/or indications (which may include instructions and illustrations) relating to at least one aspect of the usage of the depilatory article. In an advantageous the protective release layer may comprise a coating of a non-stick material. Exemplary non-stick coatings include wax, silicone, fluoropolymers such as TEFLON®, and fluorosilicones. In a preferred embodiment, the protective release layer covers at least the entire aforementioned coated region of the flexible substrate. In another preferred embodiment the protective release layer is water impermeable. In a further preferred embodiment, the protective release layer has a mean thickness of at least 85 microns, more preferably from 85 microns to 130 microns, even more preferably from 90 microns to 120 microns. In yet another preferred embodiment, the protective release layer extends beyond the coated region of the flexible substrate to provide a removal tab.

In a preferred embodiment, the depilatory articles of the present invention are packaged to prevent water loss and/or oxygen permeation. Alternatively, the depilatory articles of the present invention are packaged in water impermeable packaging. Examples of suitable packaging materials include films of EVOH; PP; PE; Nylon; foil laminates (including metalized PET; BOPP and PE), mixtures thereof, laminates thereof or multi-laminates thereof. More preferably, the packaging comprises an inert gas and even more preferably the inert gas comprises at least one of nitrogen, argon or carbon dioxide. Alternatively, the packaging comprises a partial vacuum.

A second aspect being a method of removing hair from the skin is also provided by the present invention, comprising the steps of:
(a) applying a depilatory article according to the present invention to the surface of the skin, preferably mammalian and more preferably human skin,
(b) leaving said depilatory article in contact with the skin for a period of at least I minute, preferably 2 to 10 minutes, more preferably 2 to 8 minutes,
(c) removing said depilatory article from the surface of the skin, and
(d) preferably rubbing, scraping, rinsing or wiping the surface of the skin in the area to which the depilatory article was applied.

Advantageously, the method of removing hair from the skin further comprises the step of tensioning the coated region of the depilatory article prior to applying it to the skin.

The same means used to apply tension to the coated region may be used to ensure that the depilatory article is applied to the surface of the body such that the coated region is applied under tension to the unwanted hair in order to maintain the improved handling characteristics described above. In a preferred embodiment, the tension is kept substantially constant during application of the depilatory article. The flexible nature of the flexible substrate allows the flexible substrate to conform to the surface of the body to offer improved contact between the depilatory composition and the unwanted hair. In a preferred embodiment, the tension may be at least partially, more preferably substantially completely released from the coated region after applying the depilatory article to the skin in order to improve the conformability of the depilatory article.

A third aspect being a depilatory kit is also provided by the present invention, which comprises at least one depilatory article of the present invention, packaging for said depilatory article(s), and at least one of a third component selected from:
a) a pre-treatment skin care composition which may comprise ingredients to promote skin conditioning (e.g. emollients), hair hydration or provide a skin barrier (e.g. hydrophobic materials) and intended for use prior to applying the depilatory article.
b) a post-treatment skin care composition which may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like as described herein above. The complementary post treatment skin care compositions may be leave-on or rinse-off compositions. The skin care compositions may also be designed to immediately follow application of the hair removal products. For example, a finishing composition may be applied to the same skin area to combat lingering odour and irritation caused by residual depilatory agent. The finishing composition may comprise a metal oxide (e.g., zinc oxide, aluminum oxide, and magnesium oxide) that is capable of complexing with any remaining depilatory agent remaining on the targeted skin area to reduce continued odour and subsequent skin irritation.
c) a tool to assist in the removal of hair and/or depilatory composition from the skin.
d) indications (which may include instructions and/or illustrations) relating to at least one aspect of usage of the depilatory article or another component of the kit.

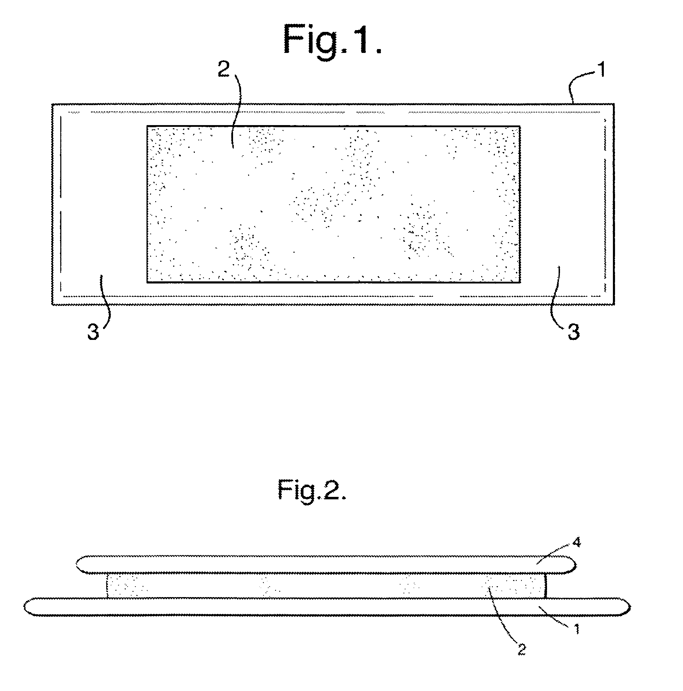

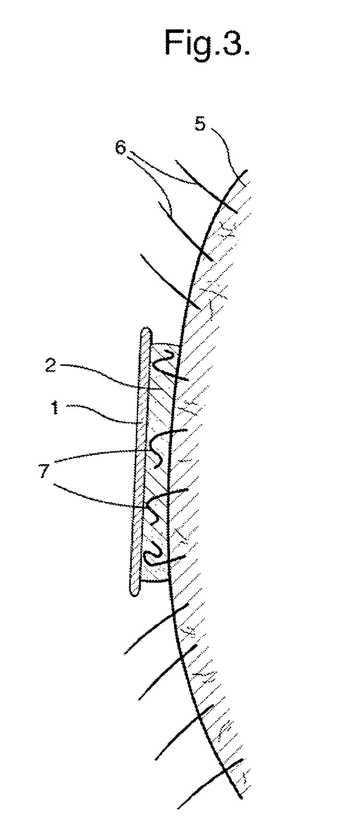

Reference is made to the figures, which disclose a non-limiting embodiment of the invention. Fig. 1 depicts a plan view of a depilatory article of the present invention, comprising a substrate(1), a depilatory composition(2) and finger-tabs(3). Fig. 2 depicts a side view of a depilatory article of the present invention, further comprising a protective release layer(4). Fig.3 depicts a side view of a depilatory article of the present invention in use, i.e. applied to keratinous tissue which comprises the skin(5), hair strands outside the depilatory composition(6) and hair strands within the depilatory composition(7).

### Example

The following example further describes and demonstrates one embodiment within the scope of the present invention. The example is given solely for the purpose of illustration and is not to be construed as a limitation of the present invention, as many variations thereof are possible.

| Formulation Ingredients | % w/w |
|---|---|
| DI water | 84.42 |
| Acrylic Acid / VP Crosspolymer (Ultrathix P- 100)¹ | 3.00 |
| Sodium Silicate (42% w/w in water) (Cognis 60)² | 2.08 |
| Calcium Hydroxide³ | 4.50 |
| Calcium Thioglycolate Trihydrate⁴ | 6.00 |

| | |
|---|---|
| 1 Ultrathix P-100 available from International Specialty Products Inc. (ISP) 2 Sodium Silicate (Cognis 60) available from Cognis 3 Calcium Hydroxide Reag. Ph. Eur. puriss. p.a. available from Sigma-Aldrich Co. 4 Calcium Thioglycolate Trihydrate 99.8% available from BRUNO BOCK Chemische Fabrik GmbH & Co. | |

A 400 ml speed mixer plastic pot was sanitized and DI water weighed in directly. The Calcium Hydroxide was added with mixing and the batch was then heated to 37 °C in a water bath for 10 minutes. The Ultrathix P-100 was then slowly added to the batch in portions over 7 min (increasing the mixing speed if required). The batch was mixed for a further 10 min (again, increasing the mixing speed as required). The batch was then cooled to room temperature using a water jacket and the Sodium Silicate was added slowly followed by the Calcium Thioglycolate. After mixing for a further 10 min to ensure full incorporation of the Calcium Thioglycolate and batch homogeneity, the batch was transferred to a thick walled 400 ml glass beaker and milled for 2 minutes using an IKA T50 (5,200 rpm).

The above formulation was disposed to a thickness of 0.3 mm, width of 3.0 cm and length of 3.5 cm on a cast HDPE 85% LLDPE 15% polymer blend film (manufactured on a Merritt-Davis casting line with LBI 85% M6030 and Exxon Mobil 15% LD2001; 23 microns in thickness and cut to be 9.5 cm in length and 3.2 cm in width) using a stencil and wiper blade, such that the area covered by the inventive formulation was centered along both the length and width of the film such as to afford a 3 cm finger tab at either end of the length of the coating of formulation. The cast HDPE 85% LLDPE 15% polymer blend film has a rigidity of 0.47 g/cm as measured on a Handle-o-Meter according to the American Standard Test Method (ASTM) D2923-06. The film also has a secant modulus at 2% strain of 3912 bar (56734 psi) according to the American Standard Test Method (ASTM)) 'Standard Test Method for Tensile Properties of Thin Plastic Sheeting D882-09' conducted on an MTS Insight1 Tensile Tester available from MTS Systems Co, Eden Prairie, MN, USA.

When used according to the method described herein, the above example provided depilatory benefit to the user.

## Claims

1. A depilatory article comprising a flexible substrate partially coated with a depilatory composition, the coating of depilatory composition forming a coated region of the depilatory article; wherein the depilatory article comprises at least two finger-tabs being substantially free of depilatory composition and positioned on substantially opposing sides of the coated region; and wherein the flexible substrate has a secant modulus at 2% strain measured according to ASTM D882-09 as disclosed herein of greater than 1379.0 bar (20,000 psi), preferably greater than 2068.4 bar (30,000 psi) and even more preferably still greater than 2757.9 bar (40,000 psi), wherein the flexible substrate comprises a polyolefin and has a thickness of from 50 µm to 15 µm.

2. A depilatory article according to claim 1, wherein said depilatory composition is disposed on said flexible substrate in an amount per unit area of the coated region of from 0.300 g/cm² to 0.001 g/cm², more preferably from 0.015 g/cm² to 0.003 g/cm², even more preferably from 0.080 g/cm² to 0.005 g/cm² and even more preferably still from 0.05 g/cm² to 0.005 g/cm².

3. A depilatory article according to either preceding claim, wherein the depilatory composition comprises a reducing agent, preferably thioglycolic acid or a thioglycolate salt, preferably present in an amount of from 0.3% to 20%, preferably from 0.8% to 15%, more preferably from 1% to 10% by weight of the depilatory composition.

4. A depilatory article according to claim 3, wherein the reducing agent comprises a divalent cation, preferably a divalent metal cation, more preferably a calcium cation.

5. A depilatory article according any preceding claim, wherein said flexible substrate has a thickness of from 40 µm to 16 µm and preferably from 30 µm to 17 µm.

6. A depilatory article according to any preceding claim, wherein at least one finger tab extends from the perimeter of the coated region by a minimum of 1 cm, preferably from 1.5 cm to 5 cm, more preferably from 2 cm to 4 cm and even more preferably from 2.5 cm to 3.5 cm, preferably wherein both finger-tabs extend from the perimeter of the coated region by a minimum of 1 cm, preferably from 1.5 cm to 5 cm, more preferably from 2 cm to 4 cm and even more preferably from 2.5 cm to 3.5 cm.

7. A depilatory article according to any preceding claim, wherein the depilatory composition comprises a base, preferably a buffering base and preferably in a concentration range of from 0.5% to 10.0%, more preferably from 1% to 8% and even more preferably 2 to 6% by weight of the depilatory composition.

8. A depilatory article according to claim 6, wherein the base comprises a divalent cation, preferably a divalent metal cation, more preferably a calcium cation, magnesium cation, zinc cation or mixtures thereof and even more preferably a calcium cation.

9. A depilatory article according to any preceding claim, wherein the depilatory composition is aqueous, preferably comprising water in an amount of at least 40%, more preferably of from 50% to 98%, even more preferably from 60% to 95% and even more preferably still from 70% to 90%, by weight of the depilatory composition.

10. A depilatory article according to any preceding claim, wherein said flexible substrate comprises high density polyethylene.

11. A depilatory article according to any preceding claim, wherein said flexible substrate is substantially planar.

12. A depilatory article according to any preceding claim, wherein said flexible substrate comprises a textured surface, preferably a microstructured surface, on at least the side upon which the depilatory composition is disposed.

13. A method of removing hair from the skin, comprising the steps of:
(a) applying a depilatory article according to any preceding claim to a surface of mammalian skin, preferably human skin,
(b) leaving said depilatory article in contact with the skin for a period of greater than 1 minute, preferably 2 to 10 minutes, more preferably 2 to 8 minutes
(c) removing said depilatory article from the surface of the skin, and
(d) preferably rubbing, scraping, rinsing or wiping the surface of the skin in the area to which the depilatory article was applied.

14. A depilatory kit, comprising:
(a) at least one depilatory article according to any of claims 1-12,
(b) optionally, at least one of a pre-treatment skin care composition, a post-treatment skin care composition and/or a tool to assist removal of hair and/or depilatory composition after use, and
(c) Packaging for said depilatory kit.

## Patentansprüche

1. Enthaarungsartikel, der ein elastisches Substrat umfasst, das teilweise mit einer Enthaarungszusammensetzung beschichtet ist, wobei die Beschichtung aus Enthaarungszusammensetzung einen beschichteten Bereich des Enthaarungsartikels bildet, wobei der Enthaarungsartikel mindestens zwei Fingeransätze umfasst, die im Wesentlichen frei von Enthaarungszusammensetzung sind und auf im Wesentlichen einander entgegengesetzt liegenden Seiten des beschichteten Bereichs angeordnet sind, und wobei das elastische Substrat einen Sekansmodul bei einer Belastung von 2 % gemessen nach ASTM D882-09 wie hierin offenbart von über 137,90 MPa (1379,0 Bar [20.000 psi]) aufweist, vorzugsweise von über 206,84 MPa (2068,4 Bar [30.000 psi]) und noch stärker bevorzugt von über 275,79 MPa (2757,9 Bar [40.000 psi]), wobei das elastische Substrat ein Polyolefin und eine Dicke von 50 µm bis 15 µm umfasst.

2. Enthaarungsartikel nach Anspruch 1, wobei die Enthaarungszusammensetzung auf dem elastischen Substrat in einer Menge pro Einheitsfläche des beschichteten Bereichs von 0,300 g/cm² bis 0,001 g/cm² abgelagert ist, stärker bevorzugt von 0,015 g/cm² bis 0,003 g/cm², noch stärker bevorzugt von 0,080 g/cm² bis 0,005 g/cm² und noch stärker bevorzugt von 0,05 g/cm² bis 0,005 g/cm².

3. Enthaarungsartikel nach einem der vorstehenden Ansprüche, wobei die Enthaarungszusammensetzung ein Reduktionsmittel umfasst, vorzugsweise Thioglycolsäure oder ein Thioglycolatsalz, vorzugsweise in einer Menge von 0,3 Gew.-% bis 20 Gew.-%, vorzugsweise von 0,8 Gew.-% bis 15 Gew.-%, stärker bevorzugt von 1 Gew.-% bis 10 Gew.-% der Enthaarungszusammensetzung.

4. Enthaarungsartikel nach Anspruch 3, wobei das Reduktionsmittel ein zweiwertiges Kation umfasst, vorzugsweise ein zweiwertiges Metallkation, stärker bevorzugt ein Kalziumkation.

5. Enthaarungsartikel nach einem der vorstehenden Ansprüche, wobei das elastische Substrat eine Dicke von 40 µm bis 16 µm aufweist und vorzugsweise von 30 µm bis 17 µm.

6. Enthaarungsartikel nach einem der vorstehenden Ansprüche, wobei sich mindestens ein Fingeransatz vom äußeren Rand des beschichteten Bereichs um mindestens 1 cm erstreckt, vorzugsweise um 1,5 cm bis 5 cm, stärker bevorzugt um 2 cm bis 4 cm und noch stärker bevorzugt um 2,5 cm bis 3,5 cm, wobei sich vorzugsweise beide Fingeransätze vom äußeren Rand des beschichteten Bereichs um mindestens 1 cm erstrecken, vorzugsweise um 1,5 cm bis 5 cm, stärker bevorzugt um 2 cm bis 4 cm und noch stärker bevorzugt um 2,5 cm bis 3,5 cm.

7. Enthaarungsartikel nach einem der vorstehenden Ansprüche, wobei die Enthaarungszusammensetzung eine Base umfasst, vorzugsweise eine Pufferbase und vorzugsweise in einer Konzentration von 0,5 Gew.-% bis 10,0 Gew.-%, stärker bevorzugt von 1 Gew.-% bis 8 Gew.-% und noch stärker bevorzugt 2 bis 6 Gew.-% der Enthaarungszusammensetzung .

8. Enthaarungsartikel nach Anspruch 6, wobei die Base ein zweiwertiges Kation umfasst, vorzugsweise ein zweiwertiges Metallkation, stärker bevorzugt ein Kalziumkation, Magnesiumkation, Zinkkation oder Mischungen davon und noch stärker bevorzugt ein Kalziumkation.

9. Enthaarungsartikel nach einem der vorstehenden Ansprüche, wobei die Enthaarungszusammensetzung wässrig ist und vorzugsweise Wasser in einer Menge von mindestens 40 Gew.-% umfasst, stärker bevorzugt von 50 Gew.-% bis 98 Gew.-%, noch stärker bevorzugt von 60 Gew.-% bis 95 Gew.-% und noch stärker bevorzugt von 70 Gew.-% bis 90 Gew.-% der Enthaarungszusammensetzung.

10. Enthaarungsartikel nach einem der vorstehenden Ansprüche, wobei das elastische Substrat hochdichtes Polyethylen umfasst.

11. Enthaarungsartikel nach einem der vorgenannten Ansprüche, wobei das elastische Substrat im Wesentlichen ebenflächig ist.

12. Enthaarungsartikel nach einem der vorstehenden Ansprüche, wobei das elastische Substrat eine strukturierte Oberfläche umfasst, vorzugsweise eine mikrostrukturierte Oberfläche, und zwar auf mindestens der Seite, auf der die Enthaarungszusammensetzung abgelagert ist.

13. Verfahren zum Entfernen von Haaren von Haut, das folgende Schritte umfasst:
(a) das Auftragen eines Enthaarungsartikels nach einem der vorstehenden Ansprüche auf eine Oberfläche einer Säugetierhaut, vorzugsweise menschlicher Haut,
(b) das Belassen des Enthaarungsartikels in Kontakt mit der Haut über einen Zeitraum von über 1 Minute, vorzugsweise 2 bis 10 Minuten, stärker bevorzugt 2 bis 8 Minuten,
(c) das Entfernen des Enthaarungsartikels von der Hautoberfläche und
(d) vorzugsweise das Abreiben, Abschaben, Abspülen oder Abwischen der Hautoberfläche in dem Bereich, in dem der Enthaarungsartikel aufgetragen wurde.

14. Enthaarungsset, das Folgendes umfasst:
(a) mindestens einen Enthaarungsartikel nach einem der Ansprüche 1 bis 12,
(b) wahlweise mindestens eine Hautvorbehandlungszusammensetzung, eine Hautnachbehandlungszusammensetzung und/oder ein Instrument zum besseren Entfernen der Haare und/oder der Enthaarungszusammensetzung nach Gebrauch und
(c) Verpackung für das Enthaarungsset.

## Revendications

1. Article dépilatoire comprenant un substrat souple partiellement revêtu d'une composition dépilatoire, le revêtement de composition dépilatoire formant une région revêtue de l'article dépilatoire ; dans lequel l'article dépilatoire comprend au moins deux languettes pour le doigt essentiellement dépourvues de composition dépilatoire et positionnées sur des côtés essentiellement opposés de la région revêtue ; et dans lequel le substrat souple a un module sécant à 2 % d'allongement, mesuré selon ASTM D882-09 comme décrit ici, supérieur à 1379,0 bar (20 000 psi), de préférence supérieur à 2068.4 bar (30 000 psi) et même plus préférablement encore supérieur à 2757,9 bar (40 000 psi), dans lequel le substrat souple comprend une polyoléfine et a une épaisseur allant de 50 µm à 15 µm.

2. Article dépilatoire selon la revendication 1, dans lequel ladite composition dépilatoire est disposée sur ledit substrat souple en une quantité par surface unitaire de la région revêtue allant de 0,300 g/cm² à 0,001 g/cm², plus préférablement de 0,015 g/cm² à 0,003 g/cm², encore plus préférablement de 0,080 g/cm² à 0,005 g/cm² et même plus préférablement encore de 0,05 g/cm² à 0,005 g/cm².

3. Article dépilatoire selon l'une ou l'autre revendication précédente, dans lequel la composition dépilatoire comprend un agent réducteur, de préférence de l'acide thioglycolique ou un sel thioglycolate, de préférence présent en une quantité de 0,3 % à 20 %, de préférence de 0,8 % à 15 %, plus préférablement de 1 % à 10 % en poids de la composition dépilatoire.

4. Article dépilatoire selon la revendication 3, dans lequel l'agent réducteur comprend un cation divalent, de préférence un cation métallique divalent, plus préférablement un cation calcium.

5. Article dépilatoire selon l'une quelconque des revendications précédentes, dans lequel le substrat souple a une épaisseur allant de 40 µm à 16 µm et de préférence de 30 µm à 17 µm.

6. Article dépilatoire selon l'une quelconque des revendications précédentes, dans lequel au moins une languette pour le doigt s'étend à partir du périmètre de la région revêtue d'un minimum de 1 cm, de préférence de 1,5 cm à 5 cm, plus préférablement de 2 cm à 4 cm et encore plus préférablement de 2,5 cm à 3,5 cm, de préférence dans lequel l'une et l'autre languettes pour le doigt s'étendent à partir du périmètre de la région revêtue d'un minimum de 1 cm, de préférence de 1,5 cm à 5 cm, plus préférablement de 2 cm à 4 cm et encore plus préférablement de 2,5 cm à 3,5 cm.

7. Article dépilatoire selon l'une quelconque des revendications précédentes, dans lequel la composition dépilatoire comprend une base, de préférence une base tampon et de préférence dans un intervalle de concentration allant de 0,5 % à 10,0 %, plus préférablement de 1 % à 8 % et encore plus préférablement 2 à 6 % en poids de la composition dépilatoire.

8. Article dépilatoire selon la revendication 6, dans lequel la base comprend un cation divalent, de préférence un cation métallique divalent, plus préférablement un cation calcium, un cation magnésium, un cation zinc ou leurs mélanges et encore plus préférablement un cation calcium.

9. Article dépilatoire selon l'une quelconque des revendications précédentes, dans lequel la composition dépilatoire est aqueuse, comprenant de préférence de l'eau en une quantité d'au moins 40 %, plus préférablement de 50 % à 98 %, encore plus préférablement de 60 % à 95 % et même plus préférablement encore de 70 % à 90 % en poids de la composition dépilatoire.

10. Article dépilatoire selon l'une quelconque des revendications précédentes, dans lequel ledit substrat souple comprend du polyéthylène à haute densité.

11. Article dépilatoire selon l'une quelconque des revendications précédentes, dans lequel ledit substrat souple est essentiellement planaire.

12. Article dépilatoire selon l'une quelconque des revendications précédentes, dans lequel ledit substrat souple comprend une surface texturée, de préférence une surface microstructurée, sur au moins le côté sur lequel la composition dépilatoire est disposée.

13. Procédé d'élimination de poils de la peau, comprenant les étapes consistant à :
(a) appliquer un article dépilatoire selon l'une quelconque des revendications précédentes sur une surface de peau de mammifère, de préférence la peau humaine,
(b) laisser ledit article dépilatoire en contact avec la peau pendant une période supérieure à 1 minute, de préférence 2 à 10 minutes, plus préférablement 2 à 8 minutes
(c) retirer ledit article dépilatoire de la surface de la peau, et
(d) de préférence frotter, racler, rincer ou essuyer la surface de la peau dans la zone sur laquelle l'article dépilatoire a été appliqué.

14. Trousse dépilatoire, comprenant :
(a) au moins un article dépilatoire selon l'une quelconque des revendications 1 à 12,
(b) facultativement, au moins un élément parmi une composition de soin de la peau de prétraitement, une composition de soin de la peau de post-traitement et/ou un outil pour faciliter le retrait des poils et/ou de la composition dépilatoire après utilisation, et
(c) un conditionnement pour ladite trousse dépilatoire.
